# EUROPÄISCHE PATENTSCHRIFT

(11) **EP 3 944 840 B1**
(45) Veröffentlichungstag und Bekanntmachung des Hinweises auf die Patenterteilung: **30.09.2026**
(21) Anmeldenummer: 21189036.3
(22) Anmeldetag: 02.08.2021
(51) Int. Cl.: A61F 5/34

(54) **ANORDNUNG ZUR AUSÜBUNG LOKALER EXTERNER DRUCKKRÄFTE AUF KÖRPERREGIONEN UND PELOTTE ZUR VERWENDUNG BEI EINER SOLCHEN ANORDNUNG**
ASSEMBLY FOR APPLYING LOCAL EXTERNAL COMPRESSION FORCES TO BODY REGIONS AND PAD FOR USE IN SUCH AN ARRANGEMENT
AGENCEMENT D'EXERCICE DES FORCES DE PRESSION EXTERNES LOCALES SUR DES ZONES CORPORELLES ET INSERTS DESTINÉS À ÊTRE UTILISÉS DANS UN TEL AGENCEMENT

(30) Priorität: 31.07.2020 DE 102020120237
(43) Veröffentlichungstag der Anmeldung: 02.02.2022
(73) Patentinhaber: FXF GmbH, 92224 Amberg (DE)
(72) Erfinder: FISCHER, Franz, 92224 Amberg (DE)
(74) Vertreter: Benninger, Johannes

(56) Entgegenhaltungen:
- EP-A1- 0 496 071
- DE-A1- 102006 048 313
- DE-A1- 102015 217 128
- DE-U1- 202007 017 518
- US-A1- 2012 078 157
- US-A1- 2015 190 269
- US-B1- 6 219 867

## Beschreibung

Die vorliegende Erfindung betrifft eine Anordnung zur Ausübung lokaler externer Druckkräfte auf Körperregionen mit den Merkmalen des Anspruchs 1, insbesondere zur Ausübung eines Oberflächendrucks auf eine definierte Körperregion eines Menschen, die insbesondere eine Narbe oder ein Narbengewebe aufweisen kann. Die Erfindung betrifft zudem eine Pelotte zur Verwendung bei einer solchen Anordnung.

Im Zusammenhang mit der Nachbehandlung und Therapie von schon verschlossenen, aber noch nicht vollständig verheilten Operationsnarben haben sich Druckverbände als vorteilhaft und günstig erwiesen. Hierbei geht es nicht nur um die Verbesserung der Wundheilung, sondern auch um die Reduzierung von narbenbedingten Taubheiten und sensorischen Einschränkungen, die bei der Zufügung von körperöffnenden Operationsschnitten unvermeidlich sind. Allerdings hat es sich gezeigt, dass eine durch Naht oder Klebung verschlossene Operationsnarbe besser verheilt und die Nervenschädigungen besser abklingen oder reduzierbar sind, wenn die Narbenregion oder das Narbengewebe mit einem äußeren Druck beaufschlagt wird.

Eine bekannte Maßnahme, um die Narbenbildung günstig zu beeinflussen und narbenbedingte Einschränkungen zu reduzieren, besteht in der Applikation eines äußeren Drucks auf eine betroffene Körperregion, etwa mittels eines Druckverbandes, eines Kompressionsstrumpfes o. dgl. Normalerweise kann die Druckbeaufschlagung dadurch unterstützt werden, dass ein Kompressionskörper unmittelbar auf die betroffene Narbenregion aufgelegt und mittels des Druckverbandes oder des Kompressionsstrumpfes dort fixiert wird. Ein solcher Kompressionskörper kann bspw. aus einem Schaumstoffmaterial bestehen.

Befindet sich die Narbe allerding in oder an einer Körperregion, die konkav geformt oder ständiger Formänderung bei Bewegungen des betroffenen Patienten unterliegt, so besteht oftmals die Schwierigkeit, den mittels des Druckverbandes und des aus Schaumstoff bestehenden Kompressionskörpers hergestellten äußeren Druck auf die Narbe und das umliegende Gewebe aufrechtzuerhalten und/oder konstant zu halten. Je nach Anwendungssituation kann es vielmehr zu Situationen kommen, wo der auf die betroffene Körperregion ausgeübte Druck zwischen ungünstig hohen Druckwerten und unzureichend geringen Druckwerten schwankt, was den angestrebten Heilungs- und Therapieeffekt stark beeinträchtigen oder sogar weitgehend zunichtemachen kann.

Eine anders geartete und allgemeinere Problemstellung kann sich in Körperregionen ergeben, die kein Narbengewebe aufweisen und generell als gesund gelten können. Insbesondere im Fußbereich lässt sich jedoch feststellen, dass für die große Bandbreite an Fußformen, Größen, Fuß- und Beinstellungen sowie hinsichtlich der individuellen Druckempfindlichkeiten und nicht zuletzt im Hinblick auf persönlich hochgradig unterschiedlich ausgeprägte Komfortempfindungen in aller Regel nur eine begrenzte Auswahl an Fußoberbekleidungen zur Verfügung gestellt werden kann.

Dies gilt nicht nur für die Sohlenkontur, die das Fußgewölbe zu stützen hat und ggf. durch eine individuell anpassbare Sohleneinlage dem jeweiligen Träger die notwendige Stützkontur zur Verfügung stellen soll, sondern auch für den Schaftbereich vieler Schuhe oder Stiefel.

So empfinden es viele Stiefelträger als problematisch, dass sich im höheren Schaftbereich Druckstellen ergeben können, die bei der ersten Anprobe nicht erkennbar oder nicht spürbar waren und die sich oftmals erst bei längerem Tragen als unkomfortabel oder sogar als schmerzhaft herausstellen. Da es jedoch kaum möglich ist, für die Vielzahl von Fuß- und Knöchelformen individuell angepasst Stiefelschäfte anzufertigen, muss sich der Träger entweder damit abfinden oder versuchen, den Druckstellen mit eingelegten Stützpolstern beizukommen.

Zur Verbesserung einer Reizstimulationswirkung für einen Träger und zur Förderung einer optimalen Durchblutung sind wiederum andere Maßnahmen sinnvoll, wie sie bspw. durch die DE 10 2015 217 128 A zur Verfügung gestellt werden sollen. Dort wird eine Pelotte zur Reizstimulation des Fußes einer Person vorgeschlagen, die an einer bestimmten Position in einer Sohle oder einer Bandage positioniert werden kann, um ihre beabsichtigte Wirkung zu entfalten. Die Pelotte umfasst eine elastische Wandung und eine darin befindliche Füllung aus Partikeln.

Die mechanischen Eigenschaften dieser bekannten Pelotte sind in einer Weise definiert, dass die unbelastete und wieder rückgestellte Pelotte mit einer bekannten unbelasteten Pelottendicke bei einer eintretenden Belastung von 10% des bestimmungsgemäßen maximalen Drucks eine Dicke von weniger als 95% der unbelasteten Pelottendicke aufweisen soll. Außerdem soll die unbelastete und wieder rückgestellte Pelotte bei einer bestimmungsgemäßen Gesamtbelastung oder Maximalbelastung eine Dicke von mehr als 20% der unbelasteten Pelottendicke aufweisen.

Weiterhin wird eine Variante der Pelotte mit einem festen, flüssigen oder gelartigen Modifikator vorgeschlagen, der sich in dem Zwischenraum der Granulatpartikel befinden soll. Als bevorzugte Modifikatoren werden solche aus Wasser, aus Alkoholen, thixotrope Substanzen, rheopexe Substanzen, organische Verbindungen, leitfähige und andere Verbindungen oder Komponenten genannt.

Ausgehend von den genannten Problemstellungen kann es als vorrangige Aufgabe der Erfindung gesehen werden, eine solchen Problemen abhelfende Anordnung, bspw. gebildet durch eine verbesserte Druckverbandanordnung und einen hierfür besser geeigneten Kompressionskörper zur Verfügung zu stellen, welche die genannten Nachteile vermeiden, insbesondere durch eine bessere Konstanthaltung einer äußeren Druckbeaufschlagung auf ein Narbengewebe und/oder eine Körperregion mit Narbengewebe oder durch eine verbesserte Druckverteilung einer Körperregion, die aufgrund des Tragens eines Oberbekleidungsstückes einer erhöhten Druckbelastung unterliegt.

Diese Aufgaben werden durch die Gegenstände der unabhängigen Ansprüche gelöst. Weitere vorteilhafte Ausgestaltungen werden durch die abhängigen Ansprüche beschrieben.

Zur Lösung der Aufgabe schlägt die Erfindung eine Anordnung zur Ausübung lokaler externer und insbesondere oberflächlich wirkender Druckkräfte auf eine definierte Körperregion oder auf mehrere definierte Körperregionen eines menschlichen Organismus mit den Merkmalen des unabhängigen Anspruchs vor. Bei dieser erfindungsgemäßen Anordnung ist vorgesehen, dass mindestens eine Pelotte definierter Elastizität und/oder Kompressibilität mittels einer ein Körperteil oder eine Körperregion mit definierter Vorspannung beaufschlagenden oder einer die Pelotte dort unter definierter Vorspannung oder Anpressdruck applizierenden Halteeinrichtung an der wenigstens einen definierten Körperregion gehalten und an diese angedrückt ist.

Bei der erfindungsgemäßen Anordnung kann insbesondere vorgesehen sein, dass die Vorspannung oder der Anpressdruck, mit der die Pelotte unter Verwendung der Halteeinrichtung an der wenigstens einen Körperregion gehalten und an diese angedrückt ist, in etwa den auf die von der Pelotte bedeckte definierte Körperregion auszuübenden externen und oberflächlich wirkenden Druckkräften entsprechen oder mit diesen in einem ursächlichen Zusammenhang stehen.

Es kann sich bei dieser Anordnung bspw. um eine Druckverbandanordnung handeln, so dass damit eine einen Oberflächendruck auf eine definierte Körperregion eines Menschen ausübende Druckverbandanordnung definiert ist, bei der mindestens eine Pelotte definierter Elastizität und/oder Kompressibilität mittels eines ein Körperteil oder eine Körperregion umschlingenden Verbandes oder einer anderen geeigneten Halteeinrichtung unter definierter Vorspannung, die in etwa dem auszuübenden Oberflächendruck entspricht oder mit diesem in einem Zusammenhang steht, an der definierten Körperregion gehalten und an diese angedrückt ist.

Die erfindungsgemäße Druckverbandanordnung ermöglicht es, sensorische Einschränkungen und andere ungünstige Effekte, die insbesondere im Zusammenhang mit der Narbenbildung und Wundheilung stehen können, zu mindern. Die Druckverbandanordnung soll nach Möglichkeit einen von den Patienten während einer erforderlichen Behandlungsdauer tolerierbaren Kompressionsdruck erzeugen, der ausreichend hoch ist, um die genannten Effekte wie insbesondere die Narbenbildung und Verheilung günstig zu beeinflussen und insbesondere um sensorische Einschränkungen und andere ungünstige Effekte zu unterdrücken oder zumindest zu reduzieren.

Weiterhin hat die Druckverbandanordnung den Vorteil, dass sie zur Erzeugung eines ausreichenden Kompressionsdrucks sehr einfach an die betroffenen Körperregionen angebracht und auch wieder entfernt werden kann. Der Kompressionsdruck kann zudem leicht variiert werden. So ist die mindestens eine Pelotte definierter Elastizität plastisch verformbar und/oder an die Oberfläche der Körperregion, an der sie angedrückt ist, anpassbar, was die gewünschte Variation des Kompressionsdrucks erlaubt.

Wahlweise jedoch kann es sich bei der erfindungsgemäßen Anordnung auch um eine Konfiguration handeln, bei der die mindestens eine Pelotte mittels einer Halteeinrichtung, die Teil einer Oberbekleidung ist oder in der Oberbekleidung integriert ist, an das mit definierten Druckkräften zu beaufschlagende Körperteil oder an die mit definierten Druckkräften zu beaufschlagende Körperregion appliziert und/oder angedrückt ist. Mit dem Begriff der Oberbekleidung kann im vorliegenden Zusammenhang insbesondere ein Schuh oder ein Stiefel, wahlweise jedoch ein Handschuh gemeint sein.

Bei einer Ausführungsvariante der erfindungsgemäßen Anordnung kann die Oberbekleidung insbesondere durch einen Schuh oder durch einen Stiefel, insbesondere durch einen Funktionsschuh oder -stiefel gebildet sein. So ist es für viele Träger und Benutzer von derartigen Funktionsschuhen eine vertraute Situation, dass bestimmte Fuß- oder Knöchelbereiche aufgrund einer krankhaften oder noch im gesunden Spektrum befindlichen Abweichung oder generell aufgrund einer von einer zu erwartenden Normkontur abweichenden Form oder Kontur nach längerem Tragen Druckstellen ausbilden, ohne dass dies beim ersten Tragen oder bei einer Anprobe unmittelbar erkennbar oder spürbar sein musste.

Dieses den Tragekomfort teilweise erheblich beeinträchtigende Phänomen tritt besonders häufig bei Ski-Stiefeln auf, die konstruktionsbedingt mit einer sehr steifen äußeren Schale ausgeführt sein müssen und die Passform sowie den Tragekomfort allenfalls über das Innenpolster gewährleisten können. Wenn nun jedoch das Innenpolster nicht optimal an die Fuß- und Knöchelform des Trägers angepasst ist, steigt die Wahrscheinlichkeit für störende und den Langzeitkomfort beeinträchtigende Druckstellen erheblich an.

Um diesen Nachteilen abzuhelfen, kann die erfindungsgemäße Pelotte an einer Stelle oder an mehreren typischen Problemstellen - dies dürften in aller Regel die inneren und äußeren Knöchelkontaktbereiche sein - im Innenpolster eingebaut, gehalten oder dort mittels der oben genannten Halteeinrichtung fixiert sein, um sich solchermaßen den exponierten Körperregionen optimal anpassen zu können, ohne dass damit eine reduzierte Stützwirkung verbunden wäre. Eine solch reduzierte Stützwirkung könnte zu einem zu lockeren Sitz des Stiefels am Fuß des Trägers führen, was jedoch durch die mindestens eine Pelotte vermieden werden kann.

Der Einbau und die Einarbeitung der erfindungsgemäßen Pelotten in das Innenpolster von Schuhen und Stiefeln kann somit die Bandbreite der Passformen verbessern, so dass mit weniger Grundformen mehr Träger zufriedengestellt werden können, ohne dass aufwändige und vergleichsweise teure Anpassungsarbeiten oder Individualisierungsmaßnahmen erforderlich werden.

Die Pelotte, die den Kompressionskörper bildet, erzeugt beim Anlegen an die zu behandelnde Körperregion einen gleichmäßigen, flächigen Kompressionsdruck, der auf das Narbengewebe und die umliegenden Körperregionen einwirken kann.

Je nach Anwendungsfall bildet die Pelotte einen definierten Druck bzw. eine Druckverteilung und einen gleichmäßigen, flächigen Kompressionsdruck aus, der auf die von Druckspitzen zu entlastenden Körperregionen wie bspw. den Knöchelbereich eines Fußes einwirken kann.

Die Pelotte unterscheidet sich damit grundlegend von allen Arten bisher verwendeter offenporiger oder geschlossenporiger Schaumstoffpolster, die allesamt die gemeinsame Eigenschaft haben, unter äußerer Druckeinwirkung nachzugeben und sich komprimieren zu lassen, und sich bei Nachlassen der Druckeinwirkung wieder elastisch zu entspannen und zurückzuformen. Allerdings lassen sich mit solchen Schaumstoffpolstern generell keine flächigen Körperregionen mit gleichmäßigem Druck beaufschlagen, zumal diese Körperregionen nicht starr sind, sondern sich mit den Bewegungen der Körperteile mitbewegen.

Durch den Einsatz der im Druckverband befindlichen und der mit diesem Druckverband zusammenwirkenden Pelotte kann der Kompressionsdruck an geeigneten Stellen des Körpers lokal erhöht und dadurch die Heilung und Narbenbildung signifikant verbessert werden. Hierfür wird die Pelotte mittels des Druckverbandes an entsprechenden Stellen des Körpers positioniert, wobei die Pelotte durch den Druckverband am vorgesehenen Ort gehalten wird und einen vergleichmäßigten Druck auf die Körperregion mit dem Narbengewebe ausüben kann.

Die Pelotte ist dadurch während der Kompressionstherapie sicher gegen Verrutschen an der betroffenen Körperregion angeordnet. Insbesondere überdeckt die Pelotte dabei das Narbengewebe und die umliegende Region und erzeugt dort eine gleichmäßige Erhöhung des Kompressionsdrucks, über den dort bereits von der Druckverbandanordnung erzeugten Kompressionsdruck hinaus.

Durch den Einsatz der im Halteverbund befindlichen und der mit der Pelotte zusammenwirkenden Halteeinrichtung können Druckspitzen an bestimmten Stellen des Körpers lokal reduziert und gleichmäßiger auf die umgebenden Bereiche, die von der Pelotte bedeckt sind, verteilt werden. Hierfür wird die Pelotte mittels der Halteeinrichtung an entsprechenden Stellen des Körpers positioniert, wobei die Pelotte durch die Halteeinrichtung am vorgesehenen Ort gehalten wird und einen vergleichmäßigten Druck auf die Körperregion(en) ausüben kann, die von Druckspitzen entlastet werden sollen.

Die Pelotte ist dadurch beim Tragen jeglicher damit ausgestatteter Oberbekleidungsstücke, sei dies ein Schuh, ein Stiefel oder ein Handschuh, zuverlässig gegen Verrutschen gesichert und an der betroffenen Körperregion angeordnet. Insbesondere überdeckt die Pelotte dabei das von Druckspitzen zu entlastende Gewebe und die umliegende Region und erzeugt dort eine gleichmäßige Reduzierung und/oder Verteilung des Kompressionsdrucks.

Die oder jede Pelotte der erfindungsgemäßen Anordnung bzw. der Druckverbandanordnung ist bevorzugt plastisch verformbar. Dies ermöglicht eine gute Anpassung der Form der jeweiligen Pelotte an die anatomische Form der betreffenden Gliedmaßen oder Körperregionen des Trägers oder Patienten, an denen die Pelotte appliziert sein soll oder an denen die Druckverbandanordnung angelegt wird. Durch eine gute Anpassung der Form der Pelotte an die Form der Gliedmaßen oder der Körperregionen, an denen die Pelotte angelegt wird, kann eine gezielte lokale Erhöhung des auf die Gliedmaßen oder die Körperregion einwirkenden Kompressionsdrucks erzielt werden. Ebenso kann bedarfsgemäß eine gezielte Verteilung und damit eine lokale Reduzierung des auf die Gliedmaßen oder die Körperregion einwirkenden Kompressionsdrucks erzielt werden.

Die Pelotte, die einen Druckkörper mit definiertem Gesamtvolumen bildet, weist konstruktionsbedingt eine definierte Elastizität auf. Die Nachgiebigkeit und Elastizität der Pelotte kann insbesondere durch Verwendung eines untervernetzten Kunststoffmaterial erreicht werden, wobei es sich hierbei sinnvollerweise um ein untervernetztes Elastomermaterial handeln kann, oder wobei die Pelotte im Wesentlichen durch ein solches untervernetztes Elastomermaterial gebildet sein kann.

Bei der erfindungsgemäßen Anordnung - dies kann bspw. auch eine Druckverbandanordnung sein - erhält die mindestens eine Pelotte definierter Elastizität eine thixotropierte Flüssigkeit, die von einer elastischen Umhüllung umschlossen ist. Hierbei ist vorgesehen, dass die thixotropierte oder thixotrope Flüssigkeit bei Temperaturen zwischen 10°C und 40°C, insbesondere zwischen 15°C und 37°C, fließfähig ist. Wahlweise kann die thixotropierte oder thixotrope Flüssigkeit bei Temperaturen zwischen 30°C und 37°C fließfähig sein.

Zudem kann eine Umhüllung der Pelotte durch ein elastisches, diffusionsgeschlossenes Material, insbesondere durch ein Elastomermaterial oder durch ein thermoplastisches Polymermaterial gebildet sein.

Die oder jede Pelotte besteht aus einer Umhüllung und einem darin eingebrachten viskoelastischen Füllmaterial. Das viskoelastische Füllmaterial kann dabei insbesondere bei Körpertemperatur (37°C) eine Fließfähigkeit aufweisen und ermöglicht solchermaßen eine individuelle Anpassung der Form der Pelotte an die Form und Kontur der Gliedmaßen und/oder Körperbereiche oder Körperregionen des Patienten, an die die Pelotte angelegt wird.

Die Umhüllung besteht zweckmäßig aus einem elastischen, diffusionsgeschlossenen Material, insbesondere aus einem thermoplastischen Polymer oder einem Elastomer, bevorzugt aus einem thermoplastischen Elastomer. Dies kann sicherstellen, dass das insbesondere flüssige Füllmaterial von der Umhüllung gehalten werden kann, ohne dass Füllmaterial bei Einwirkung eines hohen Kompressionsdrucks aus der Umhüllung austreten kann. Weiterhin beeinträchtigt die Umhüllung das viskoelastische Verhalten des Füllmaterials nicht nachteilig, so dass eine optimale Anpassung der Form der Pelotte an die Form der Gliedmaßen oder Körperregionen, an die die Pelotte angelegt wird, gewährleistet bleibt.

Ein besonders geeignetes Material für die Umhüllung ist ein thermoplastisches Polymer, wie z.B. TPU, TPA, TPC oder TPO. Dies ermöglicht eine plastische Verformung der Pelotte im angelegten Zustand bei Körpertemperatur und dadurch eine gute Anpassung der Pelottenform an die Anatomie der Gliedmaßen oder Körperregionen des Patienten, an die die Pelotte angelegt wird.

Als besonders geeignetes Elastomermaterial hat sich untervernetztes Polyurethan (PU) herausgestellt, das in einer Umhüllung aus vernetztem Polyurethanmaterial eingebettet ist. Die Umhüllung hat damit folienähnliche Eigenschaften und bildet jedenfalls eine flexible, aber nicht übermäßig dehnbare Umhüllung für das untervernetzte PU, so dass dieses unter äußerer Druckeinwirkung fließen und als hochviskose oder zähe hydraulische Flüssigkeit innerhalb der äußeren Begrenzung der Umhüllung ausweichen kann.

Grundsätzlich können sich auch andere Materialien als das genannte PU für den vorgesehenen Einsatzzweck der Pelotte eignen, doch sind es besonders die Eigenschaften des untervernetzten Polyurethans im Zusammenhang mit der Umhüllung aus vernetztem Polyurethan, die eine Pelotte mit weitgehend gleichmäßig über das gesamte Volumen verteilter Elastizität und Viskosität liefern können.

In einer bevorzugten Ausführungsform besteht das Füllmaterial aus einem Gemisch aus einem pflanzlichen oder synthetischen Bindemittel und einem mineralischen oder synthetischen Füllstoff, wobei der Füllstoff bevorzugt ausgewählt ist aus Mikrohohlkugeln, insbesondere aus Siliciumdioxid und/oder Aluminiumoxid (auch als Cenosphäre oder mircroballons bezeichnet), oder Talkum (Magnesiumsilikathydrat in Pulverform) oder einer Mischung davon.

Ein solches Gemisch zeichnet sich durch ein viskoelastisches Verhalten über einen größeren Temperaturbereich, insbesondere im Bereich zwischen Raumtemperatur (21 °C) und Körpertemperatur (37°C) aus.

Gemäß der Erfindung ist das Füllmaterial thixotrop. Dies bewirkt eine Abnahme der Viskosität infolge des auf die Pelotte einwirkenden Drucks des Kompressionsartikels. Durch die Viskositätsabnahme wird das Füllmaterial dünnflüssiger und die Pelotte kann sich noch besser der Form der Gliedmaßen oder betroffenen Körperregionen anpassen. Nach Beendigung der Kompressionstherapie und Abnahme des Kompressionsartikelsets von den betreffenden Gliedmaßen geht die Form der Pelotte wieder in ihre Ausgangsform zurück, da kein äußerer Druck mehr auf die Pelotte einwirkt.

Die Pelotte hat dabei eine Shorehärte von weniger als 17, wobei dieser Wert vorzugsweise als Shore-A gemessen oder definiert wird. Bei einer solchen Pelotte mit einer Shore-A-Härte von ca. 17 oder etwas weniger ist zwar ein Ausweichen unter Druckeinwirkung gegeben, aber das elastische Material der Pelotte ist stärker vernetzt als dies teilweise wünschenswert ist. Ein solches Material befindet sich an der Grenze der Fließfähigkeit. Für manche Einsatzzwecke ist eine solche Pelotte ideal geeignet, jedoch nicht für alle Fälle.

In der Praxis haben sich Werte für die Pelotte mit einer Shore-A-Härte von deutlich weniger als 17, vorzugsweise Werte von ca. 10 bis 15 als besonders sinnvoll für den vorgesehenen Einsatzzweck erweisen. Bei einer solchen Pelotte mit einer Shore-A-Härte von ca. 10 bis 15 oder sogar noch etwas weniger ist ein ausgeprägtes Ausweichen und Fließen unter Druckeinwirkung gegeben, da das elastische Material der Pelotte weniger stark vernetzt ist als bei der oben beschriebenen Variante (Shore-A ≈ 17) der Fall ist. Ein solches Material weist eine ausgeprägte Fließfähigkeit auf, formt sich bei nachlassendem Druck aber dennoch in die Ausgangsform zurück.

Je nach Bedarf kann jede Pelotte zumindest auf einer Seite konkave oder konvexe Bereiche aufweisen, die sinnvollerweise auf die jeweils zu therapierende Körperregion und deren Kontur abgestimmt sind. Zudem können diese konkaven oder konvexen Bereiche das Fließverhalten in günstiger bzw. in gewünschter Weise beeinflussen und verbessern.

Wie erwähnt, kann das mit der Pelotte beaufschlagte oder das unterhalb der dort fixierten Pelotte befindliche Körperteil insbesondere ein Narbengewebe sein. Zudem kann die mit der Pelotte beaufschlagte oder die unterhalb der dort fixierten Pelotte befindliche Körperregion eine Narbe aufweisen oder ein Narbengewebe enthalten.

Wahlweise kann das mit der Pelotte beaufschlagte oder das unterhalb der dort fixierten Pelotte befindliche Körperteil ein Bereich sein, bei dem der lokale Druck oder bei dem lokale Druckspitzen reduziert werden sollen. Zudem kann die mit der Pelotte beaufschlagte oder die unterhalb der dort fixierten Pelotte befindliche Körperregion ein hervorstehendes Teil sein oder bspw. hervorstehende Knochen- oder Skelettstrukturen enthalten.

Vorzugsweise ist die definierte Vorspannung, mit der die Pelotte an das Körperteil oder die Körperregion angedrückt ist, variabel und/oder durch eine veränderliche Zugspannung der Druckverbandanordnung einstellbar. Wahlweise kann die definierte Vorspannung, mit der die Pelotte an das Körperteil oder die Körperregion angedrückt ist, mittels eines Kompressionsschlauches erzeugt sein oder werden, der das Körperteil oder die Körperregion umfängt. Ein solcher Kompressionsschlauch kann bspw. Teil eines Kompressionsstrumpfes o. dgl. sein.

Außerdem ist es sinnvoll, die Pelotte in ihrer Größe und/oder in ihrem Volumen an die zu bedeckende Körperregion oder an das zu bedeckende Körperteil anzupassen bzw. jeweils angepasste Pelotten zur Verfügung zu stellen und/oder zu verwenden. So kann die Pelotte in ihrer Größe und/oder in ihrem Volumen an die zu bedeckende Narbe oder an das mit Druck zu beaufschlagende Narbengewebe angepasst sein.

Zudem schlägt die Erfindung zur Lösung der genannten Aufgabe eine Pelotte zur Verwendung in einer Druckverbandanordnung gemäß einer der zuvor beschriebenen Ausführungsvarianten zur Behandlung von Narbengewebe und/oder zur Behandlung von narbenbildungsbedingten Effekten wie sensorischen Einschränkungen, Gewebebildungsprozessen etc. vor, wobei die Pelotte definierte elastische Eigenschaften aufweist und eine thixotropierte oder thixotrope Flüssigkeit enthält, die von einer elastischen Umhüllung umschlossen ist.

Eine solche Pelotte ermöglicht eine gute Anpassung an die anatomischen Gegebenheiten von unterschiedlichen Patienten.

Das Füllmaterial der Pelotte ist bevorzugt ein Stoffgemisch aus einer ersten Komponente (A) und einer zweiten Komponente (B). Die erste Komponente (A) ist dabei ein synthetischer oder mineralischer Füllstoff und die zweite Komponente (B) ist ein Bindemittel. Die erste Komponente (A) ist bevorzugt ausgewählt aus Talkum oder Mikrohohlkugeln ("micro ballons"), insbesondere aus Siliciumdioxid oder Aluminiumoxid, oder einer Mischung davon. Die zweite Komponente (B) ist bevorzugt ein pflanzliches Bindemittel, insbesondere ein Pflanzenöl wie Rapsöl, Leinöl oder Kokosöl, oder ein synthetisches Bindemittel, wie z.B. Silikonöl.

Wie erwähnt, ist die Umhüllung der Pelotte bevorzugt aus einem diffusionsgeschlossenen Material, insbesondere aus einem thermoplastischen Polymer, bevorzugt einem Elastomer und besonders bevorzugt einem thermoplastischen Elastomer, und dient zur Aufnahme des Füllmaterials. Das diffusionsgeschlossene Material der Umhüllung verhindert dabei, dass das zähflüssige oder ölige Füllmaterial auch bei größerem Druck, der von dem zugeordneten Kompressionsartikel auf die verwendungsgemäß an einer bestimmten Körperregion eines Patienten angelegte Pelotte ausgeübt wird, aus der Umhüllung heraustreten kann.

Ferner ermöglichen die elastischen und thermoplastischen Eigenschaften des Umhüllungsmaterials eine gute Anpassung der Form der Pelotte bei Körpertemperatur an die Anatomie der Gliedmaßen oder Körperregionen, an die die Pelotte angelegt wird.

Das Füllmaterial ist bei dieser Verwendung eine thixotrope Flüssigkeit. Dadurch sind die Fließeigenschaften des Füllmaterials abhängig von dem Druck, der während der Behandlung von der zugeordneten Druckverbandanordnung auf die Pelotte ausgeübt wird. Bei zunehmendem Druck und zunehmender Dauer der Druckeinwirkung kann das Füllmaterial ausweichen, da es in gewissen Grenzen fließfähig ist; es kann sich daher an die Form und die Konturen der Gliedmaßen oder Körperregionen anpassen, an die die Pelotte verwendungsgemäß angelegt wird.

Es kann je nach Füllmaterial auch vorgesehen sein, dass bei zunehmendem Druck und zunehmender Dauer der Druckeinwirkung das Füllmaterial dünnflüssiger und dadurch fließfähiger wird, wodurch es sich wiederum optimal an die Form und die Konturen der Gliedmaßen oder Körperregionen anpassen kann, an die die Pelotte verwendungsgemäß angelegt wird.

Beide genannten Varianten gewährleisten eine optimale Druckverteilung im betroffenen Körperbereich, wo die Pelotte bevorzugt gemäß der Verwendung angelegt wird.

Durch die bevorzugte Auswahl der Materialien der Umhüllung, insbesondere deren Elastizität und plastische Verformbarkeit bei Körpertemperatur, sowie der (viskoelastischen) Fließeigenschaften des Füllmaterials können die Pelotten so angepasst werden kann, wie es die anatomischen Gegebenheiten erfordern. Gleichzeitig ermöglicht die bevorzugte Kombination von Umhüllungsmaterial und Füllmaterial, dass sich die Pelotte an die Bewegungen der jeweiligen Körperregion des Patienten anpasst und diese nicht behindert. Der Patient behält daher während des Tragens der Druckverbandanordnung und damit während der Therapie mit einer erfindungsgemäßen Druckverbandanordnung eine möglichst hohe Bewegungsfreiheit.

Zum Anlegen und Fixieren der Pelotte an der jeweiligen Körperregion eines Patienten wird die Pelotte beim verwendungsgemäßen Einsatz im vorgesehenen Bereich platziert und der Druckverband oder die sonstige Befestigung (z.B. Pflaster, Klebeverbindung etc.) wird so über der Pelotte angelegt, dass sich die Pelotte zwischen dem zu bedeckenden Narbengewebe am Körper und dem die Pelotte fixierenden Druckverband (oder sonstiger Befestigung) befindet. Die Pelotte wird dadurch von dem Druckverband (oder der sonstigen Befestigung) in einer für die Verbesserung der Wundheilung und besseren Narbenbildung etc. optimalen Position gehalten.

Im Folgenden soll ein Ausführungsbeispiel die Erfindung und ihre Vorteile anhand der beigefügten Zeichnungen näher erläutern. Die Größenverhältnisse der einzelnen Elemente zueinander in den Zeichnungen entsprechen nicht immer den realen Größenverhältnissen, da einige Formen vereinfacht und andere Formen zur besseren Veranschaulichung vergrößert im Verhältnis zu anderen Elementen dargestellt sind.
Fig. 1 zeigt eine Ausführungsvariante einer erfindungsgemäßen Druckverbandanordnung.
Fig. 2A zeigt eine Innenseite einer menschlichen Hand und die Position der oberflächlichen Arterienbögen, wie sie im Bereich der Handfläche verlaufen.
Fig. 2B zeigt eine Anwendung einer Druckverbandanordnung mit einer Pelotte, die auf die Handfläche aufgesetzt ist.

Es sei darauf hingewiesen, dass die dargestellte Ausführungsform lediglich ein Beispiel darstellt, wie die Erfindung ausgestaltet sein kann; sie soll keine abschließende Begrenzung darstellen oder definieren. Auch sind die nachfolgend beschriebenen Merkmale jeweils nicht in engem Zusammenhang mit weiteren Merkmalen des jeweiligen Ausführungsbeispiels zu verstehen, sondern können jeweils im allgemeinen Zusammenhang vorgesehen sein bzw. hierfür Verwendung finden.

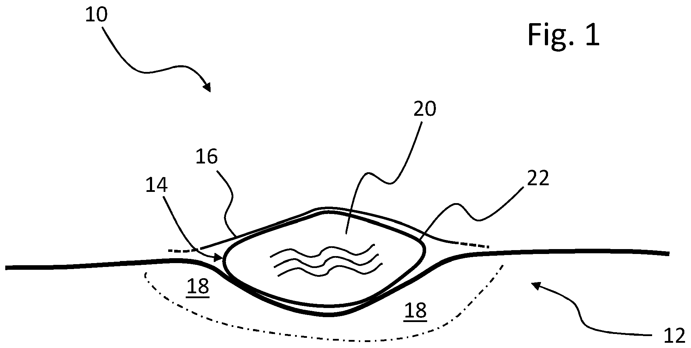

Die schematische Darstellung der Fig. 1 verdeutlicht eine Ausführungsvariante einer erfindungsgemäßen Druckverbandanordnung 10, mit der ein definierter Oberflächendruck auf eine abgegrenzte Körperregion 12 eines hier nicht näher dargestellten Menschen ausgeübt werden kann. Die erfindungsgemäße Druckverbandanordnung 10 umfasst eine Pelotte 14 definierter Elastizität und/oder Kompressibilität sowie mindestens einen, die betroffene Körperregion 12 umschlingenden Verband 16, der auf die Körperregion 12 sowie die Pelotte 14 eine definierte Vorspannung ausübt.

Die erfindungsgemäße Druckverbandanordnung 10 ermöglicht es, sensorische Einschränkungen und andere ungünstige Effekte, die insbesondere im Zusammenhang mit der Narbenbildung und Wundheilung stehen können, zu mindern. Aus diesem Grund liegt die Pelotte 14 insbesondere im Bereich einer Narbe 18 auf, die im Begriff ist abzuheilen. Die Druckverbandanordnung 10 ermöglicht es, während einer erforderlichen Behandlungsdauer einen mittels des Verbandes 16 einstellbaren Kompressionsdruck zu erzeugen, der ausreichend hoch ist, um die genannten Effekte wie insbesondere die Narbenbildung und Verheilung günstig zu beeinflussen und insbesondere um sensorische Einschränkungen und andere ungünstige Effekte zu unterdrücken oder zumindest zu reduzieren.

Die Druckverbandanordnung 10 kann sehr einfach an die betroffenen Körperregionen 12 mit den Narbenbereichen 18 angebracht und auch wieder entfernt werden. Der Kompressionsdruck kann zudem leicht variiert werden. So ist die mindestens eine Pelotte 14 definierter Elastizität plastisch verformbar und/oder an die Oberfläche der Körperregion 12, an der sie angedrückt ist, anpassbar, was die gewünschte Variation des Kompressionsdrucks erlaubt.

Die Pelotte 14, die den Kompressionskörper bildet, erzeugt beim Anlegen an die zu behandelnde Körperregion 12 einen gleichmäßigen, flächigen Kompressionsdruck, der auf das Narbengewebe 18 und die umliegenden Körperregionen 12 in gewünschter Weise einwirken kann.

Die Pelotte 14 ist während einer solchen Kompressionstherapie sicher gegen Verrutschen an der betroffenen Körperregion 12 gehalten, was durch den Verband 16 sichergestellt werden kann. Insbesondere überdeckt die Pelotte 14 dabei das Narbengewebe 18 und die umliegende Körperregion 12 und erzeugt dort eine gleichmäßige Erhöhung des Kompressionsdrucks, über den dort bereits von der Druckverbandanordnung 10 erzeugten Kompressionsdruck hinaus.

Die für die Druckverbandanordnung 10 verwendete Pelotte 14 ist plastisch verformbar. Dies ermöglicht eine gute Anpassung der Form der jeweils verwendeten Pelotte 14 an die anatomische Form der betreffenden Gliedmaßen oder Körperregionen 12 des Patienten, an dem die Druckverbandanordnung 10 angelegt wird. Durch eine gute Anpassung der Form der Pelotte 14 an die Form der Gliedmaßen oder der Körperregionen 12, an denen die Pelotte 14 angelegt wird, kann eine gezielte lokale Erhöhung des auf die Gliedmaßen oder die Körperregion 12 einwirkenden Kompressionsdrucks erzielt werden.

Bei der erfindungsgemäßen Druckverbandanordnung 10 enthält die mindestens eine Pelotte 14 definierter Elastizität eine thixotropierte Flüssigkeit 20, die von einer elastischen Umhüllung 22 umschlossen ist. Hierbei ist vorgesehen, dass die thixotropierte oder thixotrope Flüssigkeit 20 bei Temperaturen zwischen 10°C und 40°C, insbesondere zwischen 15°C und 37°C, fließfähig ist. Wahlweise kann die thixotropierte oder thixotrope Flüssigkeit 20 bei Temperaturen zwischen 30°C und 37°C fließfähig sein.

Zudem kann eine Umhüllung 22 der Pelotte 14 durch ein elastisches, diffusionsgeschlossenes Material, insbesondere durch ein Elastomermaterial oder durch ein thermoplastisches Polymermaterial gebildet sein.

Bevorzugt besteht die Pelotte 14 aus einer Umhüllung 22 und einem darin eingebrachten viskoelastischen Füllmaterial 20. Das viskoelastische Füllmaterial 20 kann dabei insbesondere bei Körpertemperatur (37°C) eine Fließfähigkeit aufweisen und ermöglicht solchermaßen eine individuelle Anpassung der Form der Pelotte 14 an die Form und Kontur der Gliedmaßen und/oder Körperbereiche oder Körperregionen 12 des Patienten, an die die Pelotte 14 angelegt wird.

Die Umhüllung 22 besteht zweckmäßig aus einem elastischen, diffusionsgeschlossenen Material, insbesondere aus einem thermoplastischen Polymer oder einem Elastomer, bevorzugt aus einem thermoplastischen Elastomer. Dies kann sicherstellen, dass das insbesondere flüssige Füllmaterial 20 von der Umhüllung 22 gehalten werden kann, ohne dass Füllmaterial 20 bei Einwirkung eines hohen Kompressionsdrucks aus der Umhüllung 22 austreten kann. Weiterhin beeinträchtigt die Umhüllung 22 das viskoelastische Verhalten des Füllmaterials 20 nicht nachteilig, so dass eine optimale Anpassung der Form der Pelotte 14 an die Form der Gliedmaßen oder Körperregionen 12, an die die Pelotte 14 angelegt wird, gewährleistet bleibt.

Ein besonders geeignetes Material für die Umhüllung 22 ist ein thermoplastisches Polymer, wie z.B. TPU, TPA, TPC oder TPO. Dies ermöglicht eine plastische Verformung der Pelotte 14 im angelegten Zustand bei Körpertemperatur und dadurch eine gute Anpassung der Pelottenform an die Anatomie der Gliedmaßen oder Körperregionen 12 des Patienten, an die die Pelotte angelegt wird.

In einer bevorzugten Ausführungsform besteht das Füllmaterial 20 aus einem Gemisch aus einem pflanzlichen oder synthetischen Bindemittel und einem mineralischen oder synthetischen Füllstoff, wobei der Füllstoff bevorzugt ausgewählt ist aus Mikrohohlkugeln, insbesondere aus Siliciumdioxid und/oder Aluminiumoxid (auch als Cenosphäre oder mircroballons bezeichnet), oder Talkum (Magnesiumsilikathydrat in Pulverform) oder einer Mischung davon.

Ein solches Gemisch zeichnet sich durch ein viskoelastisches Verhalten über einen größeren Temperaturbereich, insbesondere im Bereich zwischen Raumtemperatur (21 °C) und Körpertemperatur (37°C) aus.

Das Füllmaterial 20 ist thixotrop. Dies bewirkt eine Abnahme der Viskosität infolge des auf die Pelotte 14 einwirkenden Drucks des Kompressionsartikels. Durch die Viskositätsabnahme wird das Füllmaterial 20 dünnflüssiger und die Pelotte 14 kann sich noch besser der Form der Gliedmaßen oder betroffenen Körperregionen 12 anpassen. Nach Beendigung der Kompressionstherapie und Abnahme des Kompressionsartikelsets von den betreffenden Gliedmaßen geht die Form der Pelotte 14 wieder in ihre Ausgangsform zurück, da kein äußerer Druck mehr auf die Pelotte 14 einwirkt.

Je nach Bedarf kann die Pelotte 14 zumindest auf einer Seite konkave oder konvexe Bereiche aufweisen, die sinnvollerweise auf die jeweils zu therapierende Körperregion 12 und deren Kontur abgestimmt sind. Zudem können diese konkaven oder konvexen Bereiche das Fließverhalten in günstiger bzw. in gewünschter Weise beeinflussen und verbessern.

Wie erwähnt, kann das mit der Pelotte 14 beaufschlagte oder das unterhalb der dort fixierten Pelotte 14 befindliche Körperteil insbesondere ein Narbengewebe 18 sein.

Zum Anlegen und Fixieren der Pelotte 14 an der jeweiligen Körperregion 12 eines Patienten wird die Pelotte 14 beim verwendungsgemäßen Einsatz im vorgesehenen Bereich, d.h. z.B. auf dem Narbengewebe 18 platziert und der Druckverband 16 oder die sonstige Befestigung (z.B. Pflaster, Klebeverbindung etc.) wird so über der Pelotte 14 angelegt, wie es die Fig. 1 in schematischer Weise andeutet, wobei sich die Pelotte 14 zwischen dem zu bedeckenden Narbengewebe 18 am Körper und dem die Pelotte 14 fixierenden Druckverband 16 (oder sonstiger Befestigung) befindet. Die Pelotte 14 wird dadurch von dem Druckverband 16 (oder der sonstigen Befestigung) in einer für die Verbesserung der Wundheilung und besseren Narbenbildung etc. optimalen Position gehalten.

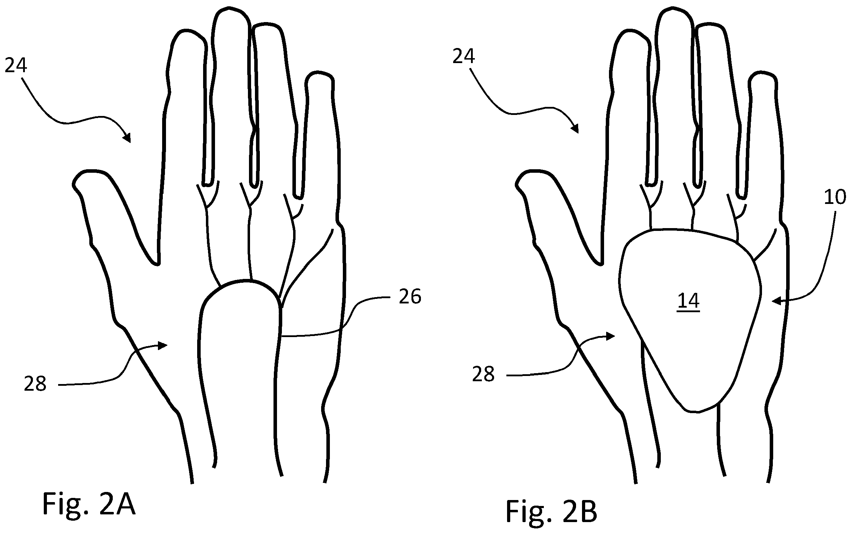

Die schematische Ansicht der Fig. 2A zeigt eine Innenseite einer menschlichen Hand 24 und die Position der oberflächlichen Arterienbögen 28, wie sie im Bereich der Handfläche 26 verlaufen.

Rechts daneben zeigt die schematische Ansicht der Fig. 2B eine Anwendung einer Druckverbandanordnung 10 gemäß Fig. 1 mit einer Pelotte 14, die auf die Handfläche 26 aufgesetzt ist. Auf die zeichnerische Darstellung des Verbandes 16 (vgl. Fig. 1) ist hier aus darstellerischen Gründen verzichtet. Da der Verband 16 oder eine anderweitige Befestigungseinrichtung jedoch zur Definition und Aufbringung der durch die Pelotte 14 auf die Handfläche 26 ausgeübten Kräfte unverzichtbar ist, ist sie in der tatsächlichen Anwendung unverzichtbar.

Wie es die Fig. 2B erkennen lässt, ist die Pelotte 14 hinsichtlich ihrer Abmessungen und Dimensionierung an die jeweilige Anwendung angepasst, hier an eine sinnvolle Abdeckung eines Teils der Handfläche 26. Die längere Achse (Längsachse) der Pelotte 14 kann somit bspw. vier bis acht Zentimeter messen, während die kürzere Achse (Querachse) demzufolge ein sinnvolles Maß zwischen drei und knapp sechs Zentimeter aufweisen kann. Eine sinnvolle Stärke einer solchen Pelotte 14, wie hier in Fig. 2B gezeigt, kann im entspannten Zustand an ihrer dicksten Stelle (d.h. in etwa mittig) zwischen eineinhalb und etwa drei Zentimeter betragen, wobei die Stärke in Richtung zu den Rändern deutlich abnimmt.

Wird die Pelotte 14 durch den Verband 16 als Teil der Druckverbandanordnung 10 gegen die Handfläche 26 gedrückt, so lässt sich die vorzugsweise konvex geformte Seite, die an der Handfläche 26 anliegt, an die Konturen der Handfläche 26 anpassen, wobei aufgrund der mechanischen Eigenschaften der Pelotte 14 der Vorteil gegeben ist, dass annähernd alle kontaktierten Bereiche der Handfläche 26 mit einem Druck in ähnlicher Größenordnung beaufschlagt werden. Um diese Druckverteilung zu verbessern, kann die von der Handfläche 26 abgewandte Seite der Pelotte 14 relativ flach geformt sein.

Wahlweise kann der in Fig. 1 in schematischer Weise angedeutete elastische Verband 16 auch durch einen Kompressionshandschuh mit definierten elastischen Eigenschaften gebildet sein, wodurch sich in Verbindung mit einer Pelotte 14 bekannter Elastizität und Verformbarkeit sehr exakt die auf die betroffene Körperregion 12 einwirkenden flächigen Druckkräfte definieren und einstellen lassen.

Die solchermaßen aufgebrachten gleichmäßigen Druckkräfte, die durch die Haut der Handfläche 26 auf den Arterienbogen 28 einwirken, können unterschiedlichen therapeutischen Zwecken dienen, so etwa bei auftretenden Neuropathien oder bei den oben erwähnten sensorischen Einschränkungen in Narbenbereichen.

Wie oben schon erwähnt, ermöglicht es der schwach vernetzte Elastomerkunststoff der Pelotte 14, der bspw. durch einen schwach vernetzten oder untervernetzten PU-Kunststoff gebildet sein kann, eine in gewissem Ausmaß fließfähige, wenn auch in ihren Eigenschaften hochviskose Kunststoffpelotte zur Verfügung zu stellen, die aufgrund ihrer hydraulischen Eigenschaften nicht nur eine Anpassung an unterschiedliche Körperformen und sich bewegende Körperbereiche liefert, sondern gleichzeitig eine annähernd gleichmäßige Druckverteilung auf die bedeckten Körperbereiche gewährleisten kann, sofern die Pelotte 14 mit definierter Andrückkraft an der zu beaufschlagenden Körperregion appliziert ist.

Die Pelotte 14 enthält eine thixotropierte Flüssigkeit 20, die von der elastischen Umhüllung 22 umschlossen ist (vgl. Fig. 1). Die thixotropierte oder thixotrope Flüssigkeit 20 ist bei Temperaturen zwischen 10°C und 40°C insbesondere zwischen 15°C und 37°C, fließfähig, wobei die Viskosität dennoch sinnvollerweise relativ hoch bleibt. Wahlweise kann die thixotropierte oder thixotrope Flüssigkeit 20 bei Temperaturen zwischen 30°C und 37°C fließfähig sein.

Zudem kann die Umhüllung 22 der Pelotte 14 durch ein elastisches, diffusionsgeschlossenes Material, insbesondere durch ein Elastomermaterial oder durch ein thermoplastisches Polymermaterial gebildet sein. Wie schon erwähnt, kann die Umhüllung 22 bspw. durch einen vernetzten PU-Kunststoff gebildet sein, so dass eine Pelotte 14 aus sortenreinem Kunststoff zur Verfügung gestellt werden kann.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

## Patentansprüche

1. Anordnung zur Ausübung lokaler externer Druckkräfte auf eine definierte Körperregion (12) oder auf mehrere definierte Körperregionen (12) eines menschlichen Organismus, umfassend mindestens eine Pelotte (14) definierter Elastizität und/oder Kompressibilität sowie eine die Pelotte (14) an die wenigstens eine Körperregion (12) applizierende Halteeinrichtung, bei welcher Anordnung die Halteeinrichtung mittels der Pelotte (14) ein Körperteil oder eine Körperregion (12) mit definierter Vorspannung beaufschlägt oder die Pelotte (14) unter definierter Vorspannung oder Anpressdruck an der wenigstens einen definierten Körperregion (12) hält und an diese andrückt, wobei die Vorspannung oder der Anpressdruck, mit der die Pelotte (14) unter Verwendung der Halteeinrichtung an der wenigstens einen Körperregion (12) gehalten und an diese angedrückt ist, in etwa den auf die von der Pelotte (14) bedeckte definierte Körperregion (12) auszuübenden externen Druckkräften entsprechen oder mit diesen in einem ursächlichen Zusammenhang stehen, und wobei die mindestens eine Pelotte (14) definierter Elastizität, die eine von einer elastischen Umhüllung (22) umschlossenen thixotropierte Flüssigkeit (20) enthält, plastisch verformbar und/oder an die Oberfläche des Körperteils oder der Körperregion (12), an das/der sie angedrückt ist, anpassbar ist, **dadurch gekennzeichnet, dass** ein Kompressionsdruck variabel ist,
wobei die thixotropierte oder thixotrope Flüssigkeit (20) bei Temperaturen zwischen 10°C und 40°C fließfähig ist, und
wobei die Pelotte (14) eine Shore-A-Härte von weniger als 17 aufweist.

2. Anordnung nach Anspruch 1, wobei die Halteeinrichtung ein Verband (16) ist, bei der die mindestens eine Pelotte (14) mittels dieses Verbandes (16), der das mit definierten Druckkräften zu beaufschlagende Körperteil oder die mit definierten Druckkräften zu beaufschlagende Körperregion (12) umschlingt, dort gehalten und/oder an das Körperteil oder an die definierte Körperregion (12) angedrückt ist.

3. Anordnung nach Anspruch 1, bei der die mindestens eine Pelotte (14) mittels einer Halteeinrichtung, bei der die Halteeinrichtung Teil einer Oberbekleidung ist oder in der Oberbekleidung integriert ist, an das mit definierten Druckkräften zu beaufschlagende Körperteil oder an die mit definierten Druckkräften zu beaufschlagende Körperregion (12) appliziert und/oder angedrückt ist.

4. Anordnung nach Anspruch 3, bei der die Oberbekleidung durch einen Schuh oder durch einen Stiefel, insbesondere durch einen Funktionsschuh oder -stiefel gebildet ist.

5. Anordnung nach einem der Ansprüche 1 bis 4, bei der die mindestens eine Pelotte (14) definierter Elastizität ein untervernetztes Kunststoffmaterial, insbesondere ein untervernetztes Elastomermaterial enthält oder im Wesentlichen durch ein solches Material gebildet ist.

6. Anordnung nach einem der Ansprüche 1 bis 5, bei der die thixotropierte oder thixotrope Flüssigkeit (20) bei Temperaturen zwischen 15°C und 37°C, fließfähig ist.

7. Anordnung nach einem der Ansprüche 5 oder 6, bei der eine Umhüllung (22) der Pelotte (14) durch ein elastisches, diffusionsgeschlossenes Material, insbesondere durch ein Elastomermaterial oder durch ein thermoplastisches Polymermaterial gebildet ist.

8. Anordnung nach einem der Ansprüche 1 bis 7, bei der die Anordnung eine Druckverbandanordnung (10) ist, bei der die definierte Vorspannung, mit der die Pelotte (14) an das Körperteil oder die Körperregion (12) angedrückt ist, variabel und/oder durch eine veränderliche Zugspannung der Druckverbandanordnung (10) einstellbar ist.

9. Anordnung nach einem der Ansprüche 1 bis 8, bei der die definierte Vorspannung, mit der die Pelotte (14) an das Körperteil oder die Körperregion (12) angedrückt ist, mittels eines Kompressionsschlauches erzeugt ist, der das Körperteil oder die Körperregion (12) umfängt, wobei die Halteeinrichtung ein Kompressionsschlauch ist.

10. Pelotte (14) zur Verwendung in einer Anordnung (10) gemäß einem der Ansprüche 1 bis 9, wobei die Pelotte (14) definierte elastische Eigenschaften aufweist und die thixotrohpierte Flüssigkeit (20) von einer elastischen Umhüllung (22) umschlossen ist, wobei die Pelotte (14) plastisch verformbar und/oder an die Oberfläche des Körperteils oder der Körperregion (12), an das/der sie angedrückt ist, anpassbar ist, wobei ein Kompressionsdruck variabel ist, wobei die thixotropierte oder thixotrope Flüssigkeit (20) bei Temperaturen zwischen 10°C und 40°C fließfähig ist, und wobei die Pelotte (14) eine Shore-A-Härte von weniger als 17 aufweist.

## Claims

1. An arrangement used for the exertion of localised external compressive forces on a defined body region (12) or on a plurality of defined body regions (12) of a human organism, the system comprising at least one pad (14) having a defined elasticity and/or compressibility, and a holding device for holding the pad (14) against the at least one body region (12), in which system the holding device applies a defined prestress to a body part or to a body region (12) by means of the pad (14), or in which the holding device holds the pad (14) and presses the pad (14) against the at least one defined body region (12) with a defined prestress or contact pressure, wherein the pad (14) is held and pressed against the at least one body region (12) by means of the holding device with a prestress or contact pressure that approximately corresponds to or is causally related to the external compressive forces to be exerted on the defined body region (12) covered by the pad (14), and wherein the at least one pad (14) having a defined elasticity and containing a thixotropic liquid (20) enclosed by an elastic sheath (22) is plastically deformable and/or adaptable to the surface of the body part or of the body region (12) against which it is pressed, **characterised in that** a compression pressure is variable,
wherein the thixotropic liquid (20) is flowable at temperatures between 10°C and 40°C, and
wherein the pad (14) has a Shore A hardness of less than 17.

2. The system according to claim 1, wherein the holding device is a bandage (16), by means of which bandage (16) the at least one pad (14) is wrapped around and held and/or pressed against the body part or the defined body region (12), to which body part or body region (12) defined compressive forces are to be applied.

3. The system according to claim 1, in which at least one pad (14) is attached to and/or pressed against the body part or the body region to which defined compressive forces are to be applied by means of a holding device, the holding device forming part of or being integrated into an outer garment (12).

4. The system according to claim 3, in which the outer garment is formed by a shoe or a boot, in particular, a functional shoe or a functional boot.

5. The system according to one of the claims 1 to 4, in which the at least one pad (14) having a defined elasticity contains an under-crosslinked plastic material, in particular, an under-crosslinked elastomeric material, or in which the at least one pad (14) is essentially formed from such a material.

6. The system according to one of the claims 1 to 5, in which the thixotropic liquid (20) is flowable at temperatures between 15°C and 37°C.

7. The system according to one of the claims 5 or 6, in which a sheath (22) for the pad (14) is formed from an elastic, diffusion-tight material, in particular, an elastomeric material or a thermoplastic polymeric material.

8. The system according to one of the claims 1 to 7, in which the system is a compression bandage system (10) in which the defined prestress pressing the pad (14) against the body part or against the body region (12) is variable and/or adjustable by means of a changeable tensile stress of the compression bandage system (10).

9. The system according to one of the claims 1 to 8, in which the defined prestress pressing the pad (14) against the body part or against the body region (12) is generated by means of a compression sleeve that surrounds the body part or body region (12), wherein the holding device is a compression sleeve.

10. A pad (14) for use in a system (10) according to one of the claims 1 to 9, wherein the pad (14) has defined elastic properties and the thixotropic liquid (20) is enclosed by an elastic sheath (22), wherein the pad (14) is plastically deformable and/or adaptable to the surface of the body part or of the body region (12) against which it is being pressed, wherein a compression pressure is variable, wherein the thixotropic liquid (20) is flowable at temperatures between 10°C and 40°C, and wherein the pad (14) has a Shore A hardness of less than 17.

## Revendications

1. Ensemble destiné à exercer des forces de pression externes locales sur une région corporelle (12) définie ou sur plusieurs régions corporelles (12) définies d'un organisme humain, comprenant au moins une pelote (14) d'élasticité et/ou de compressibilité définies ainsi qu'un dispositif de maintien appliquant ladite pelote (14) sur ladite au moins une région corporelle (12), dans lequel ensemble ledit dispositif de maintien applique, au moyen de la pelote (14), une précontrainte définie à une partie du corps ou une région corporelle (12), ou maintient la pelote (14) sous une précontrainte ou une pression de contact définie sur ladite au moins une région corporelle (12) définie et la presse contre celle-ci, dans lequel la précontrainte ou la pression de contact avec laquelle la pelote (14) est maintenue sur ladite au moins une région corporelle (12) et est pressée contre celle-ci en utilisant le dispositif de maintien correspondent à peu près aux forces de pression externes à exercer sur la région corporelle (12) définie recouverte par la pelote (14), ou y sont causalement liées, et dans lequel ladite au moins une pelote (14) d'élasticité définie qui contient un liquide thixotropé (20) enfermé par un revêtement élastique (22) est déformable plastiquement et/ou est adaptable à la surface de la partie du corps ou de la région corporelle (12) sur laquelle elle est pressée, **caractérisé par le fait qu'**une pression de compression est variable,
dans lequel le liquide thixotropé ou thixotrope (20) est fluide à des températures comprises entre 10 °C et 40 °C, et
dans lequel la pelote (14) présente une dureté Shore A inférieure à 17.

2. Ensemble selon la revendication 1, dans lequel le dispositif de maintien est un bandage (16) dans lequel ladite au moins une pelote (14) est maintenue, au moyen de ce bandage (16) qui entoure la partie du corps qui doit être soumise à des forces de pression définies ou la région corporelle (12) qui doit être soumise à des forces de pression définies, sur celle-ci, et/ou est pressée contre la partie du corps ou contre la région corporelle (12) définie.

3. Ensemble selon la revendication 1, dans lequel ladite une moins une pelote (14) est appliquée et/ou pressée contre la partie du corps devant être soumise à des forces de pression définies ou contre la région corporelle (12) devant être soumise à des forces de pression définies, au moyen d'un dispositif de maintien, dans lequel le dispositif de maintien fait partie d'un vêtement de dessus ou est intégré au vêtement de dessus.

4. Ensemble selon la revendication 3, dans lequel le vêtement de dessus est formé par une chaussure ou une botte, en particulier par une chaussure ou une botte fonctionnelle.

5. Ensemble selon l'une quelconque des revendications 1 à 4, dans lequel ladite au moins une pelote (14) d'élasticité définie contient un matériau plastique sous-réticulé, en particulier un matériau élastomère sous-réticulé, ou est constitué pour l'essentiel d'un tel matériau.

6. Ensemble selon l'une quelconque des revendications 1 à 5, dans lequel le liquide thixotropé ou thixotrope (20) est fluide à des températures comprises entre 15 °C et 37 °C.

7. Ensemble selon l'une quelconque des revendications 5 ou 6, dans lequel un revêtement (22) de la pelote (14) est constitué d'un matériau élastique et résistant à la diffusion, en particulier d'un matériau élastomère ou d'un matériau polymère thermoplastique.

8. Ensemble selon l'une quelconque des revendications 1 à 7, dans lequel ledit ensemble est un ensemble de bandage compressif (10) dans lequel la précontrainte définie avec laquelle la pelote (14) est pressée contre la partie du corps ou la région corporelle (12) est réglable de manière variable et/ou par une contrainte de traction variable de l'ensemble de bandage compressif (10).

9. Ensemble selon l'une quelconque des revendications 1 à 8, dans lequel la précontrainte définie avec laquelle la pelote (14) est pressée contre la partie du corps ou la région corporelle (12) est générée au moyen d'un tube de compression qui entoure la partie du corps ou la région corporelle (12), dans lequel le dispositif de maintien est un tube de compression.

10. Pelote (14) destinée à être utilisée dans un ensemble (10) selon l'une quelconque des revendications 1 à 9, dans laquelle la pelote (14) possède des propriétés élastiques définies et le liquide thixotropé (20) est enfermé par un revêtement élastique (22), dans laquelle la pelote (14) est plastiquement déformable et/ou adaptable à la surface de la partie du corps ou de la région corporelle (12) sur laquelle elle est pressée, dans laquelle une pression de compression est variable, dans laquelle le liquide thixotropé ou thixotrope (20) est fluide à des températures comprises entre 10 °C et 40 °C, et dans laquelle la pelote (14) présente une dureté Shore A inférieure à 17.
